# EUROPEAN PATENT SPECIFICATION

(11) **EP 3 034 049 B1**
(45) Date of publication and mention of the grant of the patent: **16.01.2019**
(21) Application number: 14847193.1
(22) Date of filing: 18.09.2014
(51) Int. Cl.: A61F 2/915, A61L 31/16, B05C 5/00, B05D 1/26

(54) **STENT, AND STENT PRODUCTION METHOD**
STENT UND STENTHERSTELLUNGSVERFAHREN
STENT, ET PROCÉDÉ DE PRODUCTION DE STENT

(30) Priority: 27.09.2013 JP 2013200869
(43) Date of publication of application: 22.06.2016
(73) Proprietor: Terumo Kabushiki Kaisha, Tokyo 151-0072 (JP)
(72) Inventor: MARUYAMA, Kazuhiro, Ashigarakami-gun Kanagawa 259-0151 (JP)
(74) Representative: Prüfer & Partner mbB Patentanwälte · Rechtsanwälte
(86) International application number: PCT/JP2014/074732
(87) International publication number: WO 2015/046022

(56) References cited:
- EP-A1- 2 444 034
- EP-A1- 2 526 902
- WO-A1-2009/065087
- WO-A1-2011/040218
- WO-A2-03/009779
- US-A1- 2003 088 307
- US-A1- 2003 088 307

## Description

### [Technical Field]

The present invention relates to a stent and a manufacturing method of a stent.

### [Background Art]

A stent is a medical device including a mesh-like cylindrical body in which annular bodies configured to include waved struts having a bending portion are sequentially juxtaposed in an axial direction so that the adjacent annular bodies are integrated with each other via a link portion. For example, the stent is applied in order to prevent a restenosis after percutaneous transluminal coronary angioplasty (PTCA) or percutaneous coronary intervention (PCI) which is used in treating myocardial infarction or angina pectoris.

In a case where a so-called bare metal stent which is not coated with a drug is applied, a restenosis rate is lower than that in a case of the PTCA or the PCI without using the stent at all. However, it has been recognized that restenosis occurs in a stent indwelling portion at a rate of approximately 20% to 30%. A major factor of restenosis is intimal hypertrophy caused by the migration and proliferation of vascular smooth muscle cells. Therefore, a drug eluting stent (DES) has been developed which prevents the restenosis by coating an outer surface of the stent with a drug for inhibiting the migration and proliferation of vascular smooth muscle cells and by eluting the drug after the stent indwells.

The drug coating is performed in such a way that a coating solution prepared by dissolving the drug and polymer into a solvent is discharged along an outer surface of a strut by using a dispenser nozzle, and thereafter, is dried and solidified (for example, refer to PTL 1).

### [Citation List]

### [Patent Literature]

[PTL 1] JP-T-2011-502723

### [Summary of Invention]

### [Technical Problem]

However, a stent is expanded and deformed when the stent indwells after reaching a target portion inside a lumen. Therefore, due to the expanding deformation, stress concentration and/or distortion occurs in a drug coating layer formed on an outer surface of a bending portion of the strut. Consequently, there is a problem in that the drug coating layer is peeled off or separated therefrom.

The present invention is made in order to solve the problem resulting from the above-described technique in the related art, and an object thereof is to provide a stent and a manufacturing method of a stent in which a drug is satisfactorily and uniformly effective by preventing the drug from being peeled off or separated due to the stress concentration and/or the distortion resulting from the expanding deformation of the stent.

### [Solution to Problem]

In order to achieve the above-described object, according to an aspect of the present invention, there is provided a stent that has an annular body configured to include a waved strut having a bending portion and multiple main strut portions. Then, only an outer surface of the main strut portion and a side surface of an end portion of the main strut portion adjacent to the bending portion are coated with a drug except the bending portion.

In order to achieve the above-described object, according to another aspect of the present invention, there is provided a manufacturing method of a stent which has a drug coating process in which the stent that has an annular body configured to include a waved strut having a bending portion and multiple main strut portions is coated with a drug. Then, in the drug coating process, only an outer surface of the main strut portion and a side surface of an end portion of the main strut portion adjacent to the bending portion are coated with the drug except the bending portion.

### [Advantageous Effects of Invention]

According to the present invention, a bending portion (portion where stress concentration and/or distortion occurs due to expanding deformation) of a strut of a stent is not coated with a drug, and has no drug coating layer formed thereon. Accordingly, it is possible to avoid the occurrence of the stress concentration and/or the distortion in the drug coating layer. In addition, in an end portion of a main strut portion which is likely to receive the influence from the bending portion, an outer surface and a side surface are coated with the drug, and a drug coating layer is formed thereon. Compared to a case where only the outer surface has the drug coating layer formed thereon, an area of the drug coating layer increases. Accordingly, the drug coating layer has improved peeling-off resistance, and the drug is provided with improved uniform efficacy. Therefore, it is possible to provide a stent and a manufacturing method of a stent in which the drug is satisfactorily and uniformly effective by preventing the drug from being peeled off or separated due to the stress concentration and/or the distortion resulting from the expanding deformation of the stent.

In the drug coating process, the stent is coated with the drug by discharging a coating solution through a nozzle unit while the nozzle unit communicating with a container for storing the coating solution obtained by dissolving the drug and a polymer into a solvent is moved along the strut. In this case, satisfactory workability can be obtained, and it is possible to easily form a coating layer having the drug loaded by the polymer.

In the drug coating process, if the nozzle unit reaches an end portion of a first main strut portion, the nozzle unit moves to an end portion of a second main strut portion without passing through the bending portion, and thereafter the nozzle unit moves toward the other end portion of the second main strut portion. In this case, it is possible to easily obtain the bending portion which has no drug coating layer formed thereon.

In the drug coating process, a coating solution supplied to the nozzle unit while the nozzle unit moves from the end portion of the first main strut portion to the end portion of the second main strut portion is held in a distal end of the nozzle unit. When the nozzle unit reaches the end portion of the second main strut portion, the coating solution held in the distal end of the nozzle unit flows down from the outer surface to the side surface of the end portion of the second main strut portion so as to coat the side surface of the end portion of the second main strut portion with the drug. In this case, satisfactory workability is achieved when the drug coating layer is formed on the side surface of the end portion of the main strut portion.

In the drug coating process, the amount of the coating solution held in the distal end of the nozzle unit is controlled by adjusting a period of time during which the nozzle unit moves from the end portion of the first main strut portion to the end portion of the second main strut portion. In this case, the amount of the coating solution is easily controlled.

In the drug coating process, moving directions of the nozzle unit are alternately and repeatedly reversed so as to increase a thickness of the drug coating layer by recoating the drug coating layer with the drug. In this case, it is possible to easily secure the required amount of the drug.

The side surface of both end portions of the main strut portion is coated with the drug. In this case, the drug coating layer has further improved peeling-off resistance, and the drug is provided with further improved uniform efficacy.

The thickness of the drug coating layer in the end portion of the main strut portion gradually decreases toward the bending portion. In this case, it is possible to minimize the occurrence of the stress concentration and/or the distortion caused by the increased thickness of the drug coating layer, thereby preventing the drug from being peeled off or separated.

When the moving directions of the nozzle unit are alternately and repeatedly reversed, a position of the nozzle unit when moving from the end portion of the first main strut portion to the end portion of the second main strut portion is changed so that the thickness of the drug coating layer gradually decreases toward the bending portion. In this case, the thickness of the drug coating layer is easily controlled, and satisfactory workability is achieved when the drug coating layer whose thickness gradually decreases toward the bending portion is formed.

The polymer is a biodegradable polymer. In this case, after the stent indwells a living body, the polymer is biodegraded, and the drug is gradually eluted. Accordingly, it is possible to reliably prevent restenosis in a stent indwelling portion. For example, the biodegradable polymer is polylactic acid, polyglycolic acid, or a copolymer of lactic acid and glycolic acid.

The annular body is arrayed at multiple locations along an axial direction of the stent, and the strut further has a link portion for integrating the adjacent annular bodies with each other. In this case, it is possible to easily obtain a stent having a desired length.

In the drug coating process, before the stent is coated with the drug, the outer surface of the strut is coated with a primer. In this case, a primer coating layer is arranged between the outer surface of the strut and the drug coating layer. Accordingly, the drug coating layer has further improved peeling-off resistance.

Other objects, features, and characteristics according to the present invention will become apparent with reference to preferred exemplary embodiments in the following description and the accompanying drawings.

### [Brief Description of Drawings]

Fig. 1 is a schematic view for describing a stent delivery system to which a stent according to an embodiment of the present invention is applied.
Fig. 2 is a plan view of the stent illustrated in Fig. 1.
Fig. 3 is an enlarged view of the stent illustrated in Fig. 1.
Fig. 4 is a plan view for describing a bending portion of the strut illustrated in Fig. 3.
Fig. 5 is a cross-sectional view for describing the bending portion of the strut illustrated in Fig. 3.
Fig. 6 is a plan view for describing a link portion of an annular body illustrated in Fig. 3.
Fig. 7 is a cross-sectional view for describing a drug coating layer illustrated in Figs. 4 to 6.
Fig. 8 is a flowchart for describing a manufacturing method of the stent illustrated in Fig. 1.
Fig. 9 is a front view for describing a coating device applied to a drug coating process illustrated in Fig. 8.
Fig. 10 is a side view of a main portion for describing the coating device applied to the drug coating process illustrated in Fig. 8.
Fig. 11 is a plan view for describing a coating route of a nozzle unit in a drug coating head illustrated in Fig. 10.
Fig. 12 is a side view for describing side surface coating performed by the nozzle unit in the drug coating head illustrated in Fig. 10.
Fig. 13 is a flowchart for describing the drug coating process.
Fig. 14 is a plan view for describing an imaging process illustrated in Fig. 13.
Fig. 15 is a flowchart for describing a coating route setting process illustrated in Fig. 13.
Fig. 16 is a flowchart for describing a first coating process illustrated in Fig. 13.
Fig. 17 is a flowchart for describing a second coating process illustrated in Fig. 13.

### [Description of Embodiments]

Hereinafter, embodiments according to the present invention will be described with reference to the drawings. In some cases, dimensional proportions in the drawings may be exaggerated and different from actual proportions for convenience of description.

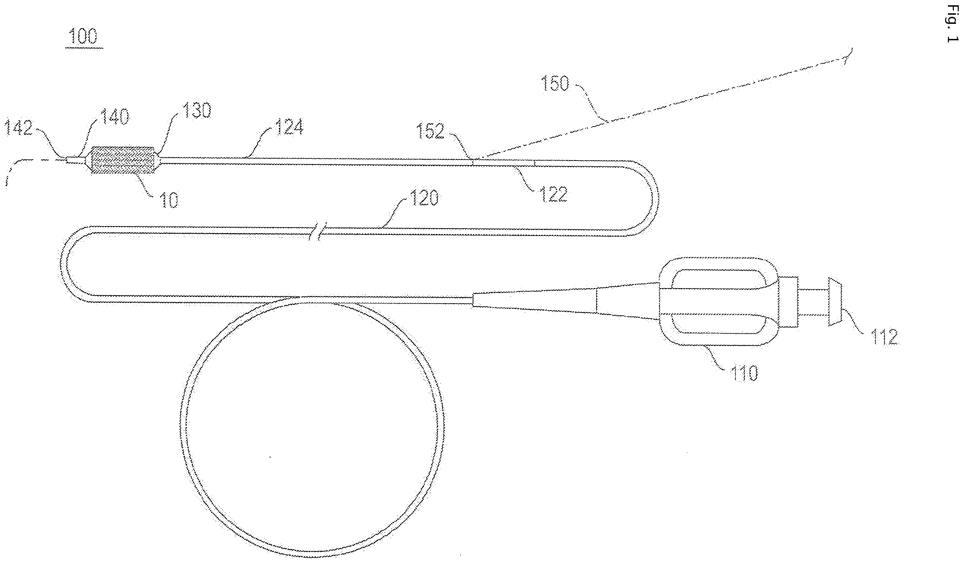

Fig. 1 is a schematic view for describing a stent delivery system to which a stent according to an embodiment of the present invention is applied.

A stent 10 according to the embodiment of the present invention includes a drug eluting stent (DES) whose outer surface is coated with a drug, and has a function as a living body indwelling device which secures a lumen by indwelling the lumen after coming into close contact with an inner surface of a stenosed portion. For example, the stent 10 is applied to a stent delivery system 100 illustrated in Fig. 1, and is utilized for treatment which aims to prevent restenosis after percutaneous transluminal coronary angioplasty (PTCA, PCI).

The stent delivery system 100 is a rapid exchange (RX) type which has a structure in which a guide wire 150 passes through only a distal portion, and has a hub 110, a proximal shaft 120, an intermediate shaft 122, a distal shaft 124, a balloon 130, and an inner tube shaft 140 in addition to the stent 10.

The hub 110 has an opening portion 112 having a lure taper formed in order to interlock an auxiliary device, and is joined to the proximal shaft 120 while a liquid-tight state is maintained. For example, the auxiliary device is an in-deflator (pressure applying device) for supplying a balloon dilating fluid. The balloon dilating fluid includes an X-ray contrast agent, a physiological salt solution, an electrolytic solution, or the like.

The proximal shaft 120 has a lumen which communicates with the opening portion 112 of the hub 110, and is joined to the intermediate shaft 122 while a liquid-tight state is maintained. The intermediate shaft 122 has a lumen which communicates with the lumen of the proximal shaft 120, and is joined to the distal shaft 124 while a liquid-tight state is maintained. The distal shaft 124 has a lumen which communicates with the lumen of the intermediate shaft 122, and is connected to the balloon 130 while a liquid-tight state is maintained. A guide wire port 152 for internally introducing the guide wire 150 is disposed in a boundary between the intermediate shaft 122 and the distal shaft 124.

The balloon 130 has the stent 10 arranged on the outer periphery, and communicates with the lumen of the distal shaft 124. The balloon 130 is arranged in a folded state (or in a deflated state) and is dilatable, and the lumen of the distal shaft 124 communicates with the opening portion 112 of the hub 110 by way of the lumen of the intermediate shaft 122 and the lumen of the proximal shaft 120. Therefore, the balloon dilating fluid introduced from the opening portion 112 of the hub 110 can reach the inside of the balloon 130. That is, the balloon dilating fluid is introduced into the balloon 130 so as to dilate the balloon 130. In this manner, the stent 10 arranged on the outer periphery of the balloon can expand so as to increase the diameter.

The inner tube shaft 140 is introduced into the distal shaft 124 from the boundary between the distal shaft 124 and the intermediate shaft 122, while a liquid-tight state is maintained. Specifically, the inner tube shaft 140 penetrates the lumen of the distal shaft 124 and the balloon. The distal portion protrudes beyond the balloon 130 while a liquid-tight state is maintained. The inner tube shaft 140 has a lumen which causes the guide wire port 152 and an opening portion 142 located on a distal portion end surface to communicate with each other. The lumen is used in order to insert the guide wire 150.

For example, the stent 10 is caused to indwell as follows by the stent delivery system 100.

First, a distal portion of the stent delivery system 100 is inserted into a lumen of a patient, and is positioned at a targeted stenosed portion while the guide wire 150 protruding beyond the opening portion 142 of the inner tube shaft 140 is moved ahead. Then, the balloon dilating fluid is introduced from the opening portion 112 of the hub 110 so as to dilate the balloon 130. The stent 10 is subjected to expansion and plastic deformation, and is brought into close contact with the stenosed portion.

Thereafter, the balloon 130 is decompressed and deflated. In this manner, the stent 10 and the balloon 130 disengage from each other so as to separate the stent 10 from the balloon 130. In this way, the stent 10 is caused to indwell the stenosed portion. Then, the stent delivery system 100 separated from the stent 10 is moved rearward, and is removed from the lumen.

Next, configuration materials or the like of each unit will be described.

The stent 10 is configured to include a biocompatible material. For example, the biocompatible material includes iron, titanium, aluminum, tin, tantalum, a tantalum alloy, platinum, a platinum alloy, gold, a gold alloy, a titanium alloy, a nickel-titanium alloy, a cobalt-based alloy, a cobalt-chromium alloy, stainless steel, a zinc-tungsten alloy, a niobium alloy, or the like.

For example, the drug (biologically active substance) for coating the outer surface of the stent 10 is at least one compound selected from a group including anticancer drugs, immunosuppressive drugs, antibiotics, anti-rheumatic drugs, anti-thrombotic drugs, HMG-CoA reductase inhibitors, ACE inhibitors, calcium antagonists, anti-hyperlipidemic drugs, integrin inhibitors, anti-allergic drugs, anti-oxidants, GP IIb/IIIa antagonists, retinoids, flavonoids, carotenoids, lipid improving drugs, DNA synthesis inhibitors, tyrosine kinase inhibitors, antiplatelet drugs, anti-inflammatory drugs, biologically-derived materials, interferon, and nitric oxide production-promoting substances.

For example, the configuration material of the hub 110 includes thermoplastic resins such as polycarbonate, polyamide, polysulfone, polyacrylate, methacrylate-butylene-styrene copolymer, and the like.

For example, the configuration material of the proximal shaft 120 includes relatively strong rigid metal materials such as stainless steel, a stainless extensible alloy, a Ni-Ti alloy, brass, and aluminum. For example, if necessary, relatively strong rigid resin materials such as polyimide, polyvinyl chloride, and polycarbonate are also applicable.

The outer diameter of the proximal shaft 120 is 0.3 mm to 3 mm, and preferably 0.5 mm to 1.5 mm. The thickness of the proximal shaft 120 is 10 µm to 150 µm, and preferably 20 µm to 100 µm. The length of the proximal shaft 120 is 300 mm to 2,000 mm, and preferably 700 mm to 1,500 mm.

For example, the configuration material of the intermediate shaft 122 and the distal shaft 124 includes a polymer material such as polyolefin, cross-linked polyolefin, polyvinyl chloride, polyamide, polyamide elastomer, polyester, polyester elastomer, polyurethane, polyurethane elastomer, fluororesin, polyimide, and the like, or a mixture of these materials. For example, polyolefin is polyethylene, polypropylene, polybutene, ethylene-propylene copolymer, ethylene-vinyl acetate copolymer, ionomer, or a mixture of two or more of these materials.

The outer diameter of the distal shaft 124 and the intermediate shaft 122 is 0.5 mm to 1.5 mm, and more preferably 0.7 mm to 1.1 mm. The thickness of the distal shaft 124 and the intermediate shaft 122 is 25 µm to 200 µm, and more preferably 50 µm to 100 µm. The length of the distal shaft 124 and the intermediate shaft 122 is 300 mm to 2,000 mm, and more preferably 300 mm to 1,500 mm.

For example, the configuration material of the balloon 130 is preferably a flexible material including a polymer material such as polyolefin, cross-linked polyolefin, polyester, polyester elastomer, polyvinyl chloride, polyurethane, polyurethane elastomer, polyphenylene sulfide, polyamide, polyamide elastomer, fluororesin, and the like, or silicone rubber, and latex rubber. For example, polyester is polyethylene terephthalate. The configuration material of the balloon 130 is not limited to a form utilizing the above-described polymer material alone. For example, it is also possible to apply a film on which the above-described polymer material is appropriately laminated.

The outer diameter of a cylindrical portion of the balloon 130 in case of being dilated is set to 1.0 mm to 10 mm, and preferably 1.0 mm to 5.0 mm. The individual length of the balloon 130 is 5 mm to 50 mm, and preferably 10 mm to 40 mm. The entire length of the balloon 130 is 10 mm to 70 mm, and preferably 15 mm to 60 mm.

For example, the configuration material of the inner tube shaft 140 is preferably a flexible material including a polymer material such as polyolefin, cross-linked polyolefin, polyvinyl chloride, polyamide, polyamide elastomer, polyester, polyester elastomer, polyurethane, polyurethane elastomer, polyimide, fluororesin, and the like, or a mixture of these materials.

The outer diameter of the inner tube shaft 140 is 0.1 mm to 1.0 mm, and preferably 0.3 mm to 0.7 mm. The thickness of the inner tube shaft 140 is 10 µm to 150 µm, and preferably 20 µm to 100 µm. The length of the inner tube shaft 140 is 100 mm to 2,000 mm, and preferably 200 mm to 1,500 mm.

Without being limited to the rapid exchange type, the stent delivery system is also applicable to an over-the-wire (OTW) type. In this case, since a structure in which the guide wire passes through to a hand operation side from the distal end is employed, the guide wire is satisfactorily replaced or operated. In addition, without being limited to a form applied to the stenosed portion appearing in coronary arteries of the heart, the stent delivery system is also applicable to the stenosed portion appearing in other blood vessels, biliary ducts, bronchial tubes, esophagi, urethrae, and the like.

Next, the stent 10 will be described in detail.

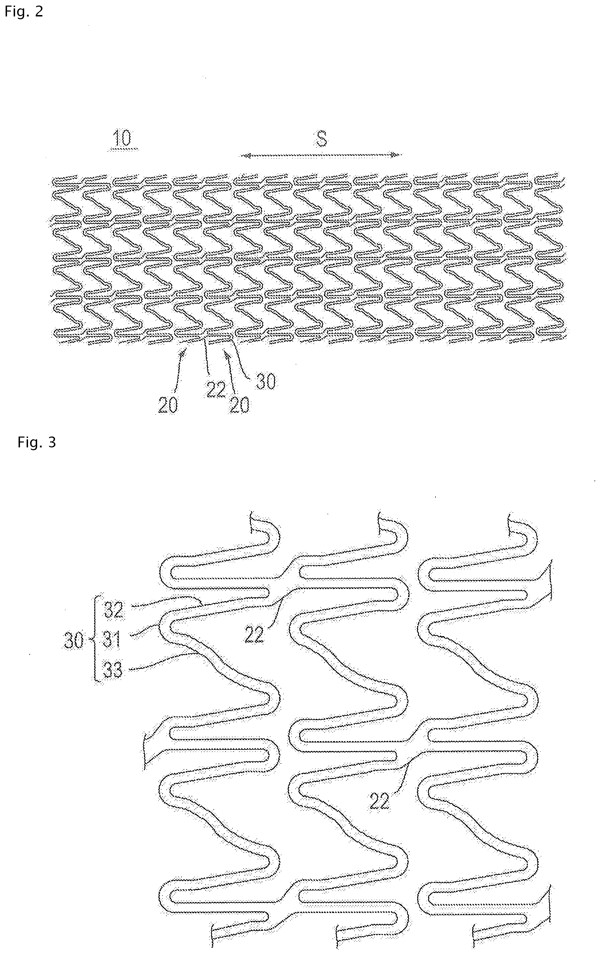

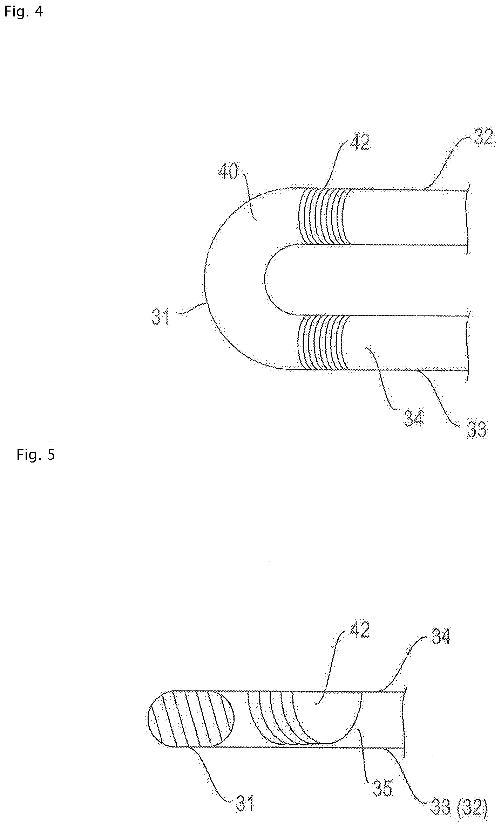

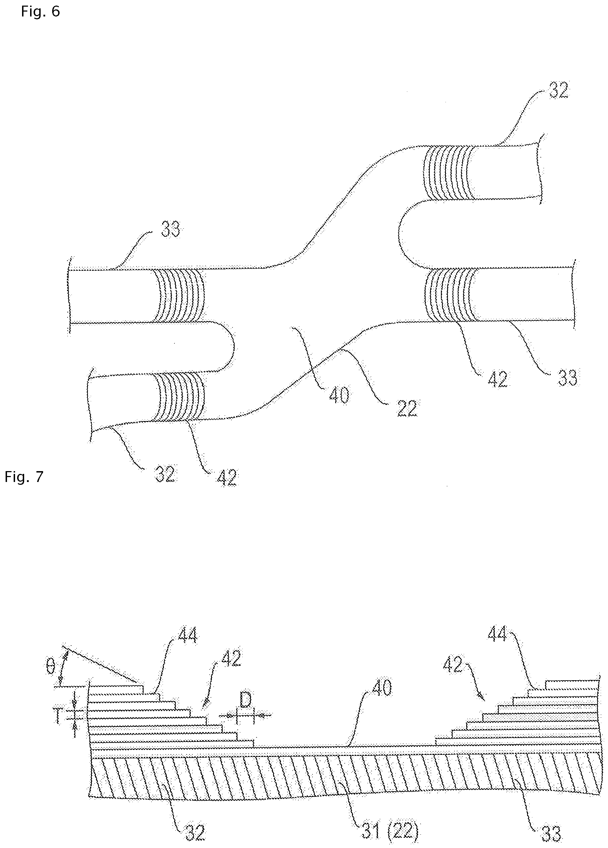

Figs. 2 and 3 are respectively a plan view and an enlarged view of the stent illustrated in Fig. 1. Figs. 4 and 5 are respectively a plan view and a cross-sectional view for describing a bending portion of a strut illustrated in Fig. 3. Fig. 6 is a plan view for describing a link portion of an annular body illustrated in Fig. 3. Fig. 7 is a cross-sectional view for describing a drug coating layer illustrated in Figs. 4 to 6. In Fig. 7, the bending portion of the strut is linearly deformed in the illustration.

As illustrated in Figs. 2 and 3, the stent 10 according to the embodiment of the present invention has an annular body 20 configured to include a strut 30. The strut 30 has multiple bending portions 31 and multiple main strut portions 32 and 33, and has a waved shape. The annular bodies 20 are sequentially juxtaposed along an axial direction S of the stent 10, and the adjacent annular bodies 20 are integrated with each other by the link portion 22. Therefore, a stent having a desired length can be easily obtained by increasing or decreasing the number of the annular bodies 20.

It is preferable that an area occupied by the strut 30 when the stent 10 does not expand in a state of being mounted on the balloon 130 is 60% to 80% of an outer peripheral area of the stent 10. The width of the strut 30 is preferably 40 µm to 150 µm, and more preferably 80 µm to 120 µm. The length of the main strut portions 32 and 33 is preferably 0.5 mm to 2.0 mm, and more preferably 0.9 mm to 1.5 mm. The diameter of the stent 10 when the stent 10 does not expand is preferably 0.8 mm to 2.5 mm, and more preferably 0.9 mm to 2.0 mm. The length of the stent 10 when the stent 10 does not expand is preferably approximately 8 mm to 40 mm.

The drug for coating the outer surface of the stent 10 is loated by a polymer so as to configure a drug coating layer 42. It is preferable that the polymer is a biodegradable polymer. In this case, after the stent 10 indwells a living body, the polymer is biodegraded, and the drug is gradually eluted. Accordingly, it is possible to reliably prevent restenosis in a stent indwelling portion.

For example, the biodegradable polymer is at least one polymer selected from a group including polyester, aliphatic polyester, polyanhydrides, polyorthoester, polycarbonate, polyphosphazenes, polyphosphate ester, polyvinyl alcohol, polypeptides, polysaccharide, protein, and cellulose, a copolymer obtained by optionally copolymerizing a monomer configuring the above-described polymer, and a mixture of the polymers and/or the copolymers. For example, aliphatic polyester is polylactic acid (PLA), polyglycolic acid (PGA), or lactic acid-glycolic acid copolymer (PLGA). Here, polylactic acid (PLA) and polycaprolactone (PCL) copolymer are employed.

As illustrated in Figs. 4 to 6, the drug coating layer 42 is arranged on an outer surface 34 of the main strut portions 32, 33 and a side surface 35 of an end portion of the main strut portions 32, 35 adjacent to the bending portion 31 and the link portion 22.

That is, the bending portion 31 and the link portion 22 (portions where stress concentration and/or distortion occurs due to expanding deformation) of the strut 30 are not coated with the drug, and have no the drug coating layer 42 formed thereon. Accordingly, it is possible to avoid the occurrence of the stress concentration and/or the distortion in the drug coating layer 42. In addition, in the end portion of the main strut portions 32, 33 which are likely to receive the influence from the bending portion 31, the outer surface 34 and the side surface 35 are coated with the drug, and the drug coating layer 42 is formed thereon. Compared to a case where only the outer surface 34 has the drug coating layer 42 formed thereon, an area of the drug coating layer 42 increases. Accordingly, the drug coating layer 42 has improved peeling-off resistance, and the drug is provided with improved uniform efficacy. Therefore, while the drug is satisfactorily and uniformly effective, it is possible to prevent the drug from being peeled off or separated due to the stress concentration and/or the distortion resulting from the expanding deformation of the stent 10.

In addition, the drug coating layer 42 is not disposed on a rear side surface of the strut 30 and a side surface other than the side surface 35 of the end portion of the above-described main strut portions 32, 33. The reason is that in case where the stent 10 indwells the blood vessel, the stent 10 does not interfere with the proliferation of endothelial cells and that the stent is encased early in blood vessel tissues. If the drug coating layer 42 is also disposed on the rear side surface of the strut 30 and the side surface other than the side surface 35 of the end portion of the main strut portions 32, 33, in a case where the stent 10 indwells the blood vessel, the stent 10 interferes with the proliferation of the endothelial cells. Consequently, there is a possibility that the stent may be disengaged therefrom.

In addition, the drug coating layer 42 is formed on the side surface 35 (refer to Fig. 5) of both end portions of the main strut portions 32, 33. Accordingly, the drug coating layer 42 has further improved peeling-off resistance, and the drug is provided with further improved uniform efficacy. If necessary, the drug coating layer 42 can also be formed on only the side surface 35 of one end portion of the main strut portions 32, 33.

A primer coating layer 40 is arranged between the drug coating layer 42 and the outer surface of the stent 10. A primer configuring the primer coating layer 40 is selected in view of adhesion to the polymer included in the drug coating layer 42 and adhesion to the outer surface of the stent 10. The presence of the primer coating layer 40 allows the drug coating layer 42 to have improved peeling-off resistance. If necessary, the primer coating layer 40 can also be omitted.

As illustrated in Fig. 7, the thickness of the drug coating layer 42 in the end portion of the main strut portions 32, 33 gradually decreases stepwise toward the bending portion 31 (and the link portion 22). Therefore, even in a case where the thickness of the drug coating layer 42 is increased, the thickness of the drug coating layer 42 located near the bending portion 31 and the link portion 22 is thin. Thus, it is possible to minimize the occurrence of the stress concentration and/or the distortion caused by the increased thickness of the drug coating layer 42, and it is possible to prevent the drug from being peeled off or separated. Accordingly, it is possible to easily secure the required amount of the drug.

A tilting angle θ of the gradually decreased portion in the drug coating layer 42 is smaller than 90 degrees, preferably 1 degree to 60 degrees, and more preferably 1 degree to 45 degrees. For example, in a case where the tilting angle θ is smaller than 1 degree, an advantageous effect in preventing the drug removal is obtained over a wide range. However, the amount of the drug for coating is reduced. In addition, if the tilting angle θ exceeds 60 degrees, there is a possibility that the advantageous effect in preventing the drug removal may be lowered.

As will be described later, the drug coating layer 42 is formed by being recoated with a coating solution prepared in such a way that the drug and the polymer are dissolved in a solvent. The length of a recoating layer 44 is sequentially changed, thereby gradually decreasing the thickness of the drug coating layer 42. A thickness T of the recoating layer 44 is preferably 1 µm to 5 µm. The thickness T of the recoating layer 44 is not limited to a form in which the thickness is always identical. The number of layers in the recoating layer 44 is preferably 2 to 50. A length difference D between the adjacent recoating layers 44 is preferably 1 µm to 1,000 µm. The length difference D is not limited to a form in which the length difference is always identical.

Next, a manufacturing method of the stent 10 will be described.

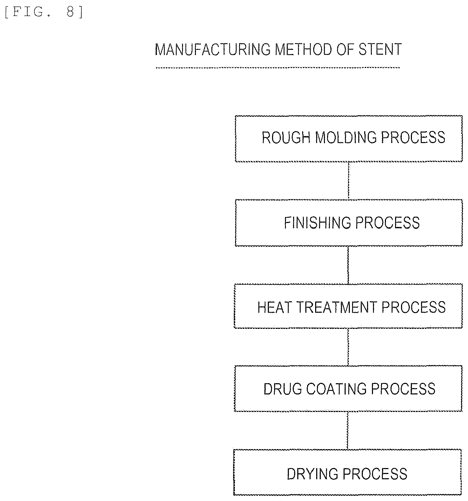

Fig. 8 is a flowchart for describing the manufacturing method of the stent illustrated in Fig. 1.

As illustrated in Fig. 8, the manufacturing method of the stent 10 has a rough molding process, a finishing process, a heat treatment process, a drug coating process, and a drying process.

In the rough molding process, a portion corresponding to a cavity portion of the stent is removed from a metal pipe body which is a stent material, thereby forming an annular body configured to include the strut and a link portion for integrating the adjacent annular bodies with each other. The portion corresponding to the cavity portion of the stent is removed by appropriately applying an etching method using masking and chemicals called photo-fabrication, an electrical discharge machining method using a mold, a cutting method, or the like. For example, the cutting method is mechanical polishing or laser cutting.

For example, in the finishing process, an edge of the strut is removed by applying chemical polishing or electrolytic polishing, thereby finishing the strut so as to have a smooth surface.

In the heat treatment process, in order to improve the flexibility and plasticity of the stent, annealing work is carried out. This enables the stent to satisfactorily indwell the curved blood vessel, and minimizes physical stimulation given to the intravascular wall. Accordingly, it is possible to reduce factors of restenosis. In the annealing work, it is preferable to gradually cool the stent after the stent is heated to 900°C to 1,200°C under an inert gas atmosphere so that an oxide film is not formed on the stent surface. For example, the inert gas is mixed gas between nitrogen and hydrogen. If necessary, the heat treatment process can also be appropriately omitted.

In the drug coating process, the stent is coated with the primer and the drug. Drug coating is performed on the outer surface 34 of the main strut portions 32, 33 and the side surface 35 of the end portion of the main strut portions 32, 35 adjacent to the bending portion 31 and the link portion 22 except the bending portion 31 (refer to Figs. 4 to 6). The drug is dissolved in a solvent together with the polymer, and is utilized in a form of a coating solution. For example, the solvent is acetone, ethanol, chloroform, or tetrahydrofuran.

In the drying process, the solvent is evaporated, and the drug coating layer 42 configured to include the drug and the polymer is formed, thereby manufacturing the stent 10.

In the manufactured stent 10, the bending portion 31 and the link portion 22 (portions where stress concentration and/or distortion occurs due to expanding deformation) of the strut 30 are not coated with the drug, and the drug coating layer 42 is not formed therein. On the other hand, the drug coating layer 42 is formed in the end portion of the main strut portions 32, 33 which is likely to receive the influence from the bending portion 31 in such a way that the outer surface 34 and the side surface 35 are coated with the drug. Therefore, as described above, in the manufactured stent 10, the drug is satisfactorily and uniformly effective, and it is possible to prevent the drug from being peeled off or separated due to the stress concentration and/or the distortion resulting from the expanding deformation of the stent 10.

Next, a coating device applied to the drug coating process and the drug coating process will be sequentially described in detail.

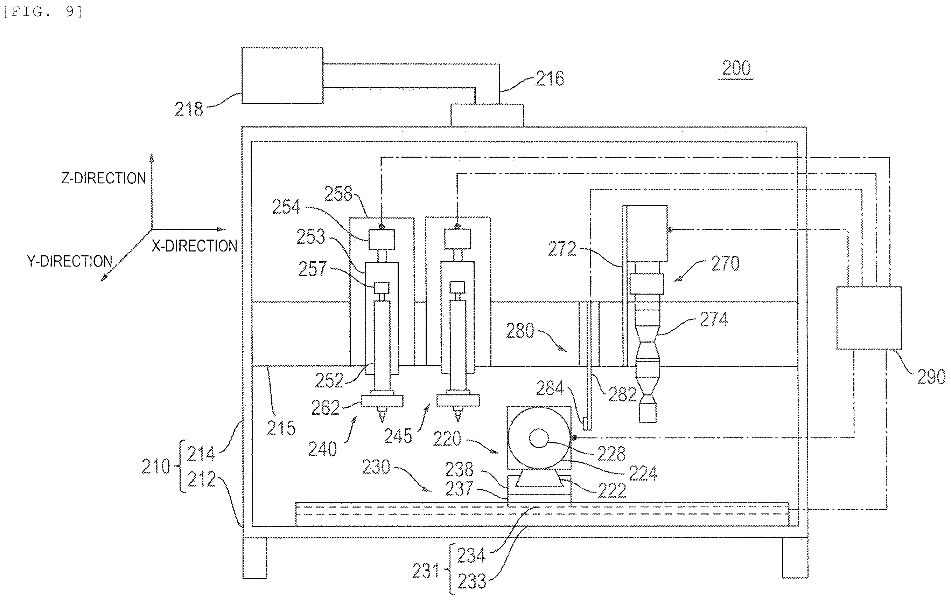

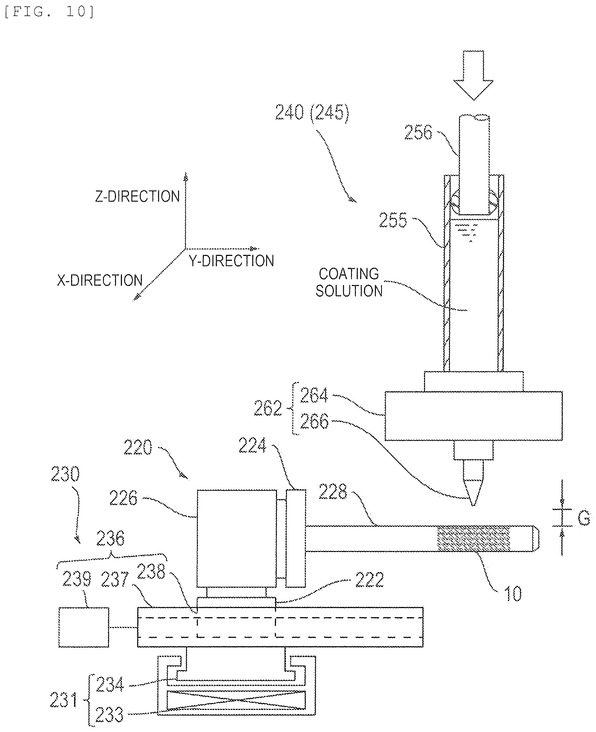

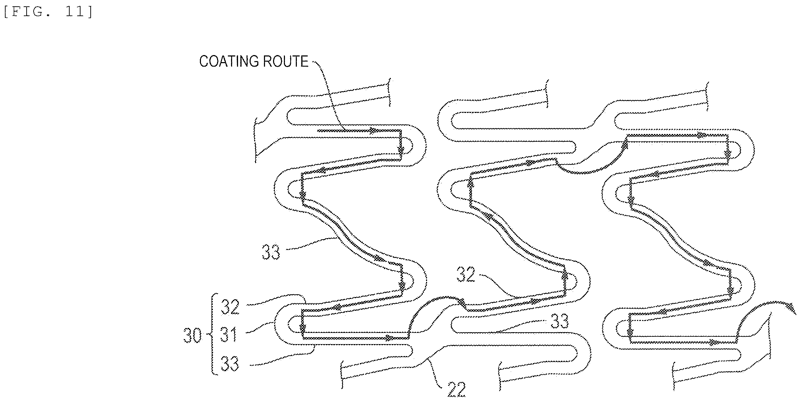

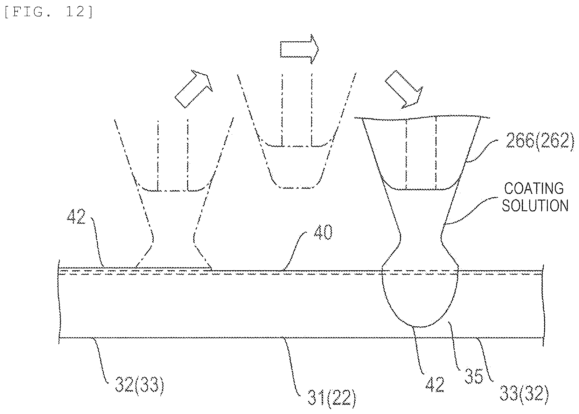

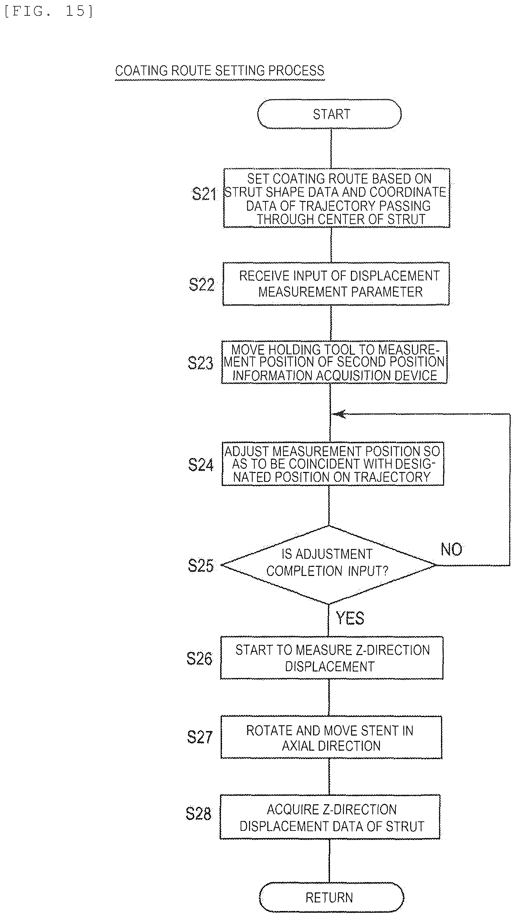

Figs. 9 and 10 are respectively a front view and a side view of a main portion for describing the coating device applied to the drug coating process illustrated in Fig. 8. Figs. 11 and 12 are respectively a plan view for describing a coating route of a nozzle unit in a drug coating head illustrated in Fig. 10, and a side view for describing side surface coating performed by the nozzle unit. In Fig. 15, the bending portion of the strut is linearly deformed in the illustration.

A coating device 200 has a chamber 210, a holding tool 220, a moving device 230, coating heads 240, 245, a first position information acquisition device 270, a second position information acquisition device 280, and a control unit 290.

The chamber 210 has a base 212, a main frame 214 arranged on the base 212, and a duct 216 interlocked to a top portion. The main frame 214 is covered with a transparent synthetic resin plate from the outer surface, and brings the inside of the chamber 210 into an airtight state. An air conditioning device 218 is interlocked to the duct 216. The air conditioning device 218 supplies temperature and humidity-controlled air to the chamber 210. Therefore, coating conditions can be made constant by maintaining the inside of the chamber 210 in a state of constant temperature and humidity. The reference numeral 215 represents a support frame which is laterally installed in the main frame 214.

The holding tool 220 is arranged at the bottom inside the chamber 210, is used in order to hold the stent 10, and has a base portion 222, a chucking portion 224, a motor 226, and a mandrel 228.

The base portion 222 is placed on the moving device 230, and is movable in an X-Y direction as will be described later. The chucking portion 224 and the motor 226 are arranged on the base portion 222. The chucking portion 224 is used in order to chuck the proximal end of the mandrel 228. The motor 226 is configured so that the chucking portion 224 can be rotated forward and rearward. The mandrel 228 has an outer periphery configured so that the stent 10 is attachable and detachable. Therefore, the holding tool 220 enables the stent 10 mounted on the mandrel 228 to rotate forward and rearward, and to move in an X-direction and a Y-direction.

It is preferable that the outer diameter of the mandrel 228 is substantially equal to or slightly larger than the inner diameter of the stent 10. In order to increase a contrast ratio between the strut 30 and the cavity portion in the stent 10, it is preferable to coat the mandrel 228 with a black paint which absorbs light. It is preferable that a concave portion for generating a gap between an outer peripheral surface of the mandrel 228 and a lower surface of the strut 30 of the stent 10 when the stent 10 is mounted on the mandrel 228 is formed on the outer peripheral surface of the mandrel 228. In this case, when the upper portion of the strut 30 is coated with the primer solution and the coating solution, it is possible to prevent the primer solution and the coating solution from intruding into a portion between the surface of the mandrel 228 and the inner surface of the stent 10. Accordingly, the primer coating layer and the drug coating layer are provided with uniform thickness, thereby improving convenience in the work. It is preferable that the mandrel 228 is replaceable. In this case, it is possible to deal with the stents 10 having various inner diameters by preparing the mandrels 228 having different outer diameter sizes.

The moving device 230 is used in order to move the holding tool 220 in the X-Y direction, and has an X-direction moving mechanism 231 and a Y-direction moving mechanism 236.

The X-direction moving mechanism 231 has a traveling rail 233 which extends in the X-direction and which has a linear motor-type drive source, and an X-direction moving table 234 which moves along the traveling rail 233. The Y-direction moving mechanism 236 has a traveling rail 237 which extends in the Y-direction, a Y-direction moving table 238 which moves along the traveling rail 237, and a motor 239 which drives the Y-direction moving table 238. The traveling rail 237 is placed on the X-direction moving table 234, and the base portion 222 of the holding tool 220 is placed on the Y-direction moving table 238.

The coating head 240 is arranged in an intermediate portion inside the chamber 210, and is used in order to coat the coating solution prepared by dissolving the drug and the polymer in the solvent. The coating head 240 has a dispenser 252, a vertical table 253, a bracket 258, and a nozzle unit 262 (refer to Fig. 10).

The dispenser 252 has a cylinder portion 255, a piston portion 256, and a drive unit 257, and is attached to the vertical table 253. The vertical table 253 is attached to the support frame 215 of the chamber 210 via the bracket 258, and is configured so that a screw feeding mechanism driven by the motor 254 enables the dispenser 252 to move in the Z-direction.

The cylinder portion 255 is a container for storing the coating solution, and is attached to the vertical table 253. The piston portion 256 is arranged inside the cylinder portion 255 so as to be slidable. For example, the drive unit 257 has a motor or a hydraulic pressure mechanism, and is configured to be capable of pressing the piston portion 256 by using predetermined force.

The nozzle unit 262 communicates with the cylinder portion 255, and has an attachment member 264 and a nozzle 266. The attachment member 264 is arranged in a lower end of the cylinder portion 255, and is used in order to interlock the nozzle 266 to the cylinder portion 255.

The outer diameter of the distal end of the nozzle 266 is preferably 10 µm to 1,000 µm. The inner diameter of the distal end of the nozzle 266 is preferably 1 µm to 500 µm, and more preferably 5 µm to 250 µm. For example, in a case where the inner diameter of the distal end of the nozzle 266 is smaller than 5 µm, the coating solution cannot smoothly flow out. In addition, great pressure is required to discharge the coating solution. In a case where the inner diameter exceeds 250 µm, there is a possibility that the coating solution cannot be smoothly used in the coating. In order to prevent the discharged coating solution from adhering to the inner surface of the nozzle 266, it is preferable to minimize the uneven surface by means of polishing.

The viscosity of the coating solution is preferably 0.1 cp to 10 cp, and more preferably 1.0 cp to 4.0 cp. For example, if the viscosity of the coating solution is higher than an upper limit of the above-described range, in some cases, discharge pressure of the coating solution becomes excessively high, or the coating solution cannot be discharged from the nozzle 266. In addition, if the viscosity is lower than a lower limit of the range, a portion of the discharged coating solution falls from the surface of the stent 10 (strut 30). Consequently, it is difficult to form a uniform coating layer.

In order to quantitatively discharge the coating solution under satisfactory control (in order to quantitatively, accurately, and reliably adjust the amount of the drug solution), a gap G between the nozzle 266 and the surface of the stent 10 (strut 30) is preferably 0.1 µm to 200 µm, and more preferably 1 µm to 100 µm. For example, if the gap G is larger than the upper limit of the range, there is a possibility that the discharging of the coating solution may be interrupted. If the gap G is smaller than the lower limit of the range, there is a possibility that the coating solution may fall from the surface of the stent 10 (strut 30).

The coating head 245 is arranged in the intermediate portion inside the chamber 210, and is used in order to perform coating of the primer solution. Except that the cylinder portion 255 stores the primer solution, the coating head 245 is substantially the same as the drug coating head 240, and thus, description thereof will be omitted.

The first position information acquisition device 270 is imaging means disposed in order to acquire position information in the X-Y direction in the rectangular coordinate system on the surface of the stent 10 (strut 30), and is attached to the support frame 215 via the bracket 272. The first position information acquisition device 270 has a camera unit 274 and a line sensor unit arranged so as to extend in the axial direction of the stent 10. The line sensor unit is used in order to scan the surface of the stent 10 in synchronization with the rotation of the stent 10 attached to the mandrel 228 of the holding tool 220, in order to acquire image data on the surface of the stent 10, and in order to transmit the image data to the control unit 290.

The second position information acquisition device 280 is Z-direction displacement measurement means disposed in order to acquire position information in the Z-direction in the rectangular coordinate system on the surface of the stent 10 (strut 30), is fixed to the lower end of the bracket 282 attached to the support frame 215, and has a laser displacement sensor 284. The laser displacement sensor 284 is a vertical sensor for measuring the Z-direction displacement of the strut 30, and is used in order to perform scanning along the trajectory passing through the center of the strut 30 while rotating the stent 10 forward and rearward, in order to acquire Z-direction displacement data of the entire stent 10, and in order to transmit the Z-direction displacement data to the control unit 290. Although not particularly limited, a measurement start point is set to a coating start position, for example.

The control unit 290 is arranged outside the chamber 210. For example, the control unit 290 has a microprocessor for controlling the above-described respective units or for performing various arithmetic processes in accordance with a program, a memory for storing various settings or data items, a monitor for displaying various settings or data items, and a keyboard for inputting various settings or data items. The control unit 290 is used in order to control the holding tool 220, the moving device 230, the coating heads 240, 245, the first position information acquisition device 270, and the second position information acquisition device 280.

For example, the arithmetic processes are performed in order to acquire the X-Y direction position information, in order to acquire the Z-direction position information, and in order to set a coating route used by the nozzle 266.

In the process for acquiring the X-Y direction position information, based on the result that the strut 30 is brighter and the cavity portion is darker, image data on the surface of the stent 10 which is acquired from the first position information acquisition device 270 is binarized so as to have suitable brightness. In this manner, the image data is separated into the strut 30 and the cavity portion, then is converted into X-Y coordinates of the strut 30, that is, the X-Y direction position information in the rectangular coordinate system, and is stored in the memory. The obtained X-Y direction position information is used in order to calculate coordinates on the trajectory passing through the center of the strut 30, and the obtained coordinates of the trajectory are stored in the memory. When coating using the coating solution is performed, it is essential to perform the coating without being separated from the strut 30. Accordingly, it is very important to identify the center of the strut 30.

In the process for acquiring the Z-direction position information, the Z-direction displacement data of the entire stent 10 which is acquired from the second position information acquisition device 280 is converted into the Z-direction position information in the rectangular coordinate system on the surface of the strut 30, and is stored in the memory. To be more exact, the strut 30 has no smooth surface, and has an uneven surface. Therefore, in order to quantitatively and accurately perform coating using the coating solution, based on the Z-direction position information, it is necessary to control the distal end of the nozzle 266 so as to move exactly parallel to the surface of the strut 30 and to perform the coating using a predetermined amount of the coating solution.

In the process for setting the coating route used by the nozzle 266, settings for performing continuous coating along the strut 30 are calculated by utilizing the X-Y direction position information and the Z-direction position information in the rectangular coordinate system of the strut 30 (the main strut portions 32, 33, the bending portion 31, and the link portion 22).

For example, as illustrated in Fig. 11, the coating route of the coating solution is set so as to continuously coat the main strut portions 32, 33 of the strut 30 with the coating solution and avoid the portions (the bending portion 31 and the link portion 22 of the strut 30) where stress concentration and/or distortion occurs due to expanding deformation. The primer coating layer has a thin thickness, and has satisfactory peeling-off resistance with respect to the strut 30. Accordingly, the coating route of the primer solution is set so as to continuously coat the entire strut 30 with the primer solution.

It is preferable that the coating route has no overlapping section. However, in some cases, it is difficult to set the stent having the struts 30 complicatedly intersecting each other so as to have no overlapping section. In this case, moving speed in the overlapping section is caused to be faster than moving speed in the non-overlapping section. In this manner, it is possible to decrease a difference between the coating thickness in the overlapping section and the coating thickness in the non-overlapping section.

The movement along the coating route can be repeated multiple times. In this case, for example, the movement and the reverse movement along the coating route (reversing the movement directions) are alternately repeated, thereby increasing the thickness of the drug coating layer. Accordingly, it is possible to easily secure the required amount of the drug.

Next, the drug coating process will be described in detail.

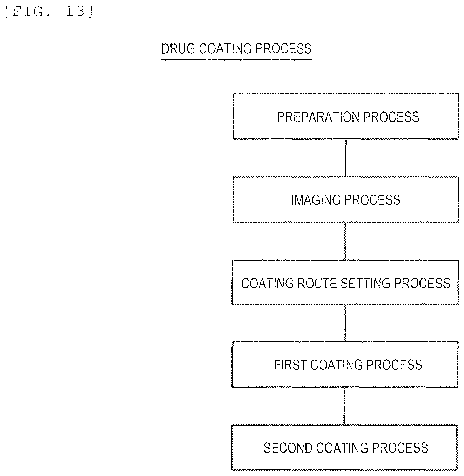

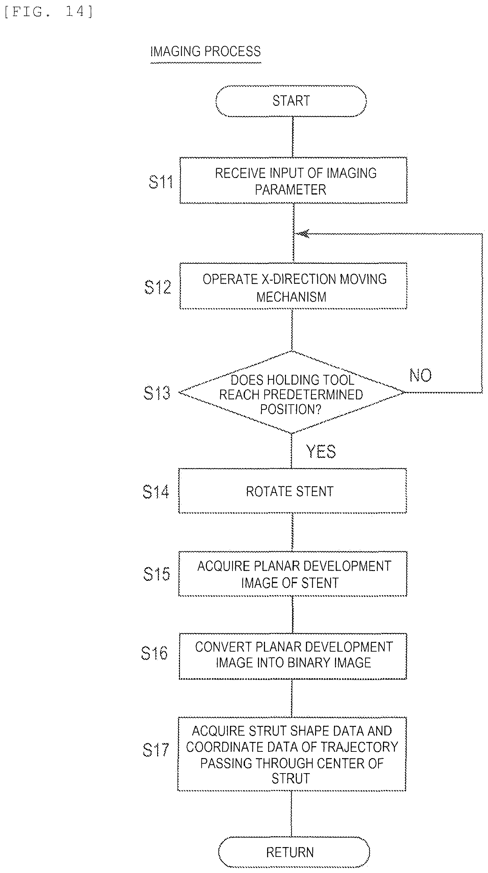

Fig. 13 is a flowchart for describing the drug coating process illustrated in Fig. 8. Figs. 14, 15, 16 and 17 are respectively flowcharts for describing an imaging process, a coating route setting process, a first coating process, and a second coating process which are illustrated in Fig. 13.

As illustrated in Fig. 13, the drug coating process has a preparation process, the imaging process, the coating route setting process, the first coating process, and the second coating process.

In the preparation process, the air conditioning device 218 is operated so as to bring the inside of the chamber 210 of the coating device 200 into a state of constant temperature and humidity. Then, the drug coating head 240 and the primer coating head 245 are attached to the support frame 215 of the chamber 210 via the vertical table 253 and the bracket 258. In addition, after being mounted on the mandrel 228, the stent 10 is positioned at a predetermined position by being attached to the chucking portion 224 of the holding tool 220 located at a standby position.

Next, the imaging process will be described with reference to Fig. 14.

First, the control unit 290 receives an input of an imaging parameter, and stores the input imaging parameter in the memory (Step S11). For example, the imaging parameter is input by an operator of the coating device 200 using a keyboard, and includes the rotation speed of the mandrel 228, the number of imaging lines obtained by the line sensor unit of the first position information acquisition device 270, the width of the imaging line, and the imaging speed.

The control unit 290 operates the X-direction moving mechanism 231 (Step S12). This causes the holding tool 220 to move along the traveling rail 233 from the standby position to a predetermined position below the first position information acquisition device 270. After confirming that the holding tool 220 reaches the predetermined position (Step S13: Yes), the control unit 290 operates the motor 226 of the holding tool 220, and rotates the mandrel 228 (stent 10) (Step S14).

The line sensor unit of the first position information acquisition device 270 scans the surface of the stent 10, and images surface patterns (Step S15). The scaned image is synthesized based on the imaging parameter, and is stored in the memory of the control unit 290 as a planar development image. If necessary, the planar development image can be output to a monitor so as to be visually confirmable.

The control unit 290 converts the planar development image of the stent 10 into a black and white binary image using a predetermined threshold value (Step S16), extracts an image of the strut 30, calculates shape data of the strut 30, and acquires coordinate data of the trajectory passing through the center of the strut 30 by performing a thinning process on the width of the strut 30 (Step S17).

Next, the coating route setting process will be described with reference to Fig. 15.

Based on the acquired shape data of the strut 30 and the acquired coordinate data of the trajectory passing through the center of the strut 30, the control unit 290 sets the coating route in the first coating process and the coating route in the second coating process (Step S21). The coating route in the first coating process is generated so that the entire strut 30 can be continuously coated and the overlapping section can be minimized. The coating route in the second coating process is generated so as to avoid the portions (the bending portion 31 and the link portion 22 of the strut 30) where stress concentration and/or distortion occurs due to expanding deformation (refer to Fig. 11).

The control unit 290 receives an input of a displacement measurement parameter, and stores the input displacement measurement parameter in the memory (Step S22). For example, the displacement measurement parameter is input by the operator of the coating device 200 using the keyboard, and includes a measurement start position, a measurement direction, measurement speed, and a measurement interval which are used by the second position information acquisition device 280.

The control unit 290 operates the motor 239 of the Y-direction moving mechanism 236, and moves the holding tool 220 (mandrel 228) to the measurement position used by the second position information acquisition device 280 (Step S23). For example, the operator visually adjusts the stent 10 mounted on the mandrel 228 and the measurement position of the second position information acquisition device 280 so that the measurement position of the second position information acquisition device 280 is coincident with the designated position on the trajectory (Step S24).

For example, if the operator of the coating device 200 inputs adjustment completion by using the keyboard (Step S25: Yes), the control unit 290 commands the second position information acquisition device 280 to start measurement of the Z-direction displacement in the strut 30 (Step S26), causes the motor 226 to repeatedly rotate forward and rearward, and causes the motor 239 to repeatedly move in the axial direction. This causes the stent 10 to repeatedly rotate and move in the axial direction (Step S27).

The second position information acquisition device 280 moves along the trajectory passing through the center of the strut 30, acquires the Z-direction displacement data of the strut 30 of the entire stent 10, and transmits the Z-direction displacement data to the control unit 290 (Step S28). The Z-direction displacement data is converted into Z-direction position information in the rectangular coordinate system on the surface of the strut 30, and is stored in the memory together with the coordinates of the center trajectory.

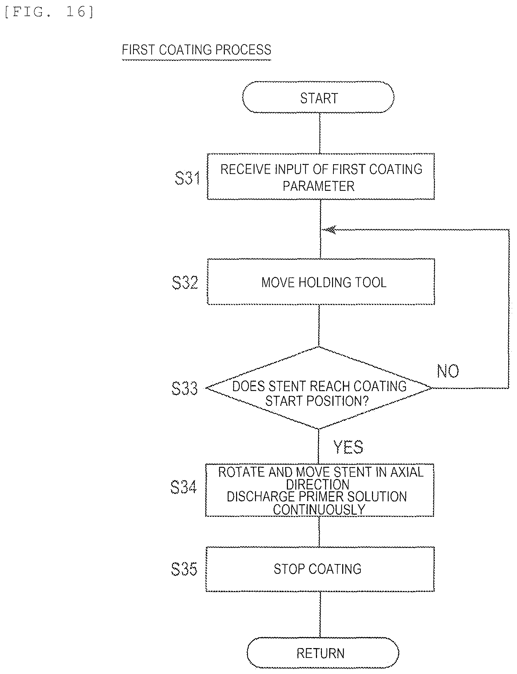

Next, the first coating process for forming the primer coating layer will be described with reference to Fig. 16.

The control unit 290 receives an input of a first coating parameter, and stores the input first coating parameter in the memory (Step S31). For example, the first coating parameter is input by the operator of the coating device 200 using the keyboard, and includes the rotation speed and the axial moving speed of the stent 10, selection of the primer coating head 245, and the discharge speed of the coating head 245 (nozzle unit 262) .

The control unit 290 commands the X-direction moving mechanism 231 to move the holding tool 220 (Step S32). This causes the stent 10 mounted on the mandrel 228 of the holding tool 220 to move to the coating start position below the coating head 245.

If the stent 10 reaches the coating start position (Step S33: Yes), the stent 10 is rotated and moved in the axial direction. The primer solution is continuously discharged from the nozzle unit 262 of the coating head 245 (Step S34). At this time, the control unit 290 commands the motor 226 to rotate forward and rearward, and commands the motor 239 to move in the axial direction so as to move the stent 10 in the X-axis direction and the Y-axis direction, in accordance with the designated parameter. The control unit 290 commands the motor 254 to cause the coating head 245 to move in the Z-axis direction. Then, if the coating of the entire strut 30 is completed once along the predetermined coating route by the coating head 245, the coating is stopped (Step S35) .

As described above, in the first coating process, the outer surface of the strut 30 is coated with the primer (primer solution) before being coated with the drug. Accordingly, the drug coating layer is provided with improved peeling-off resistance. If necessary, the first coating process can also be omitted.

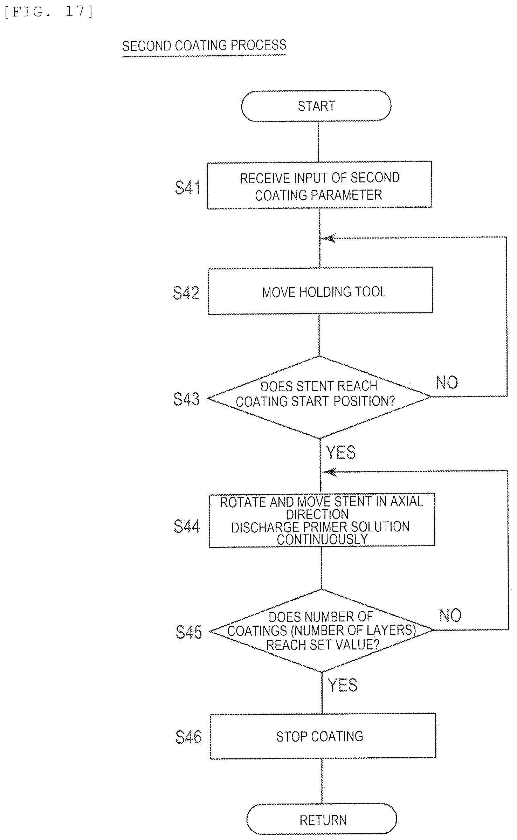

Next, the second coating process for forming the drug coating layer will be described with reference to Fig. 17.

The control unit 290 receives an input of a second coating parameter, and stores the input second coating parameter in the memory (Step S41). For example, the second coating parameter is input by the operator of the coating device 200 using the keyboard, and includes the rotation speed and the axial moving speed of the stent 10, selection of the drug coating head 240, the discharge speed of the coating head 240 (nozzle unit 262), and the number of coating processes (number of layers).

The control unit 290 commands the X-direction moving mechanism 231 to move the holding tool 220 (Step S42). This causes the stent 10 mounted on the mandrel 228 of the holding tool 220 to move to the coating start position below the coating head 240.

If the stent 10 reaches the coating start position (Step S43: Yes), the stent 10 is rotated and moved in the axial direction, and the coating solution is continuously discharged from the nozzle unit 262 of the coating head 240 (Step S44). At this time, the control unit 290 commands the motor 226 to rotate forward and rearward, and commands the motor 239 to move in the axial direction so as to move the stent 10 in the X-axis direction and the Y-axis direction, in accordance with the designated parameter. The control unit 290 commands the motor 254 to cause the coating head 240 to move in the Z-axis direction.

As a result, the coating head 240 performs coating using the coating solution while moving along the predetermined coating route (refer to Fig. 11) in the second coating process. That is, the main strut portions 32, 33 of the strut 30 are continuously coated with the coating solution except the portions (the bending portion 31 and the link portion 22 of the strut 30) where stress concentration and/or distortion occurs due to expanding deformation.

Specifically, if the nozzle unit 262 reaches the end portion of the main strut portion 32 (33), the nozzle unit 262 does not pass through the bending portion 31 (or the link portion 22) adjacent to the end portion, and moves to the end portion of the main strut portion 32 (33) adjacent to the other end portion of the bending portion 31 (or the link portion 22). Thereafter, the nozzle unit 262 moves toward the other end portion of the main strut portion 32 (33). This can avoid a possibility that the bending portion 31 (or the link portion 22) may be coated with the drug. Therefore, it is possible to easily obtain the bending portion 31 (or the link portion 22) on which the drug coating layer 42 is not formed.

Then, without being limited to the coating route (refer to Fig. 11) for performing coating using the coating solution as described above, even when the other coating route is set, it is possible to easily obtain the bending portion 31 (or the link portion 22) on which the drug coating layer 42 is not formed.

In addition, the coating solution to be supplied to the nozzle unit 262 while the nozzle unit 262 moves from the end portion of the main strut portion 32 (33) to the end portion of the other main strut portion 32 (33) is held in the distal end of the nozzle unit 262. Therefore, when the nozzle unit 262 reaches the end portion of the other main strut portion 32 (33), the coating solution held in the distal end of the nozzle unit 262 flows down to the side surface from the outer surface of the end portion of the other main strut portion 32 (33). In this manner, the side surface 35 of the end portion of the other main strut portion 32 (33) is coated with the drug (refer to Fig. 12). Accordingly, satisfactory workability is achieved when the drug coating layer 42 is formed on the side surface 35 of the end portion 32 (33) of the main strut portion.

The amount of the coating solution held in the distal end of the nozzle unit 262 can be controlled by adjusting the time (moving speed) during which the nozzle unit 262 moves from the end portion of the main strut portion 32 (33) to the end portion of the other main strut portion 32 (33). In this case, the amount of the coating solution is easily controlled. In addition, the amount of the coating solution held in the distal end of the nozzle unit 262 can be controlled by pressing the piston portion 256 storing the coating solution, changing the force, and adjusting discharge pressure of the coating solution.

If the number of coating processes (number of layers) reaches a set value by alternately repeating the movement and the reverse movement along the coating route (reversing the movement directions) (Step S45: Yes), the coating is stopped (Step S46). If the holding tool 220 is moved to the standby position by the X-direction moving mechanism 231, the mandrel 228 is detached from the holding tool 220. Then, the stent 10 (refer to Fig. 7) on which the primer coating layer 40 and the drug coating layer 42 are formed is detached from the mandrel 228.

The movement directions of the nozzle unit 262 are alternately and repeatedly reversed, thereby performing recoating of the drug and increasing the thickness of the drug coating layer 42. Accordingly, it is possible to easily secure the required amount of the drug. In addition, in this manner, the drug coating layer 42 is formed on the side surface of both end portions of the main strut portion 32 (33). Accordingly, the drug coating layer 42 has further improved peeling-off resistance, and the drug is provided with further improved uniform efficacy.

When the moving directions of the nozzle unit 262 are alternately and repeatedly reversed, a position of the nozzle unit 262 when moving from the end portion of the main strut portion 32 (33) to the end portion of the other main strut portion 32 (33) is changed. In this manner, the thickness of the drug coating layer 42 in the end portion of the main strut portion 32 (33) can be caused to gradually decrease toward the bending portion 31 (and the link portion 22). In this case, it is possible to minimize the occurrence of stress concentration and/or distortion caused by the increased thickness of the drug coating layer 42, thereby preventing the drug from being peeled off or separated. In addition, the thickness is easily controlled, and satisfactory workability is achieved when the drug coating layer 42 whose thickness gradually decreases toward the bending portion 31 (and the link portion 22) is formed.

As described above, in the second coating process, the coating solution is discharged from the nozzle unit 262 while the nozzle unit 262 communicating with the cylinder portion 255 storing the coating solution is moved along the strut portion 30. In this manner, coating is performed by using the drug. Accordingly, satisfactory workability can be obtained, and it is possible to easily form the drug coating layer 42.

As described above, according to the present embodiment, the bending portion (portion where stress concentration and/or distortion occurs due to expanding deformation) of the strut of the manufactured stent is not coated with the drug, and has no drug coating layer formed thereon. Accordingly, it is possible to avoid the occurrence of the stress concentration and/or the distortion in the drug coating layer. In addition, in the end portion of the main strut portion which is likely to receive the influence from the bending portion, the outer surface and the side surface are coated with the drug, and the drug coating layer is formed thereon. Compared to a case where only the outer surface has the drug coating layer formed thereon, an area of the drug coating layer increases. Accordingly, the drug coating layer has improved peeling-off resistance, and the drug is provided with improved uniform efficacy. Therefore, it is possible to provide the stent and the manufacturing method of the stent in which the drug is satisfactorily and uniformly effective by preventing the drug from being peeled off or separated due to the stress concentration and/or the distortion resulting from the expanding deformation of the stent.

Without being limited to the above-described embodiment, the present invention can be modified in various ways within the scope described in Claims. For example, the coating head storing a different coating solution in the cylinder portion may be installed at multiple locations. While the coating heads are switched therebetween, the coating solution can also be used for recoating. In this case, according to a form in which the coating solution varies depending on the drug concentration, the drug concentration in the thickness direction of the drug coating layer is changed. In addition, according to a form in which the coating solution varies depending on a type of drugs, the type of drugs is changed in accordance with a position in the thickness direction of the drug coating layer. In this manner, it is possible to obtain composite efficacy.

Furthermore, a method of forming the drug coating layer 42 is not limited to the above-described embodiment. For example, a spray method or an ink jet method may be employed.

### [Reference Signs List]

- 10: STENT,
- 20: ANNULAR BODY,
- 22: LINK PORTION,
- 30: STRUT,
- 31: BENDING PORTION,
- 32,: 33 MAIN STRUT PORTION,
- 34: OUTER SURFACE,
- 35: SIDE SURFACE,
- 40: PRIMER COATING LAYER,
- 42: DRUG COATING LAYER,
- 44: RECOATING LAYER,
- 100: STENT DELIVERY SYSTEM,
- 110: HUB,
- 112: OPENING PORTION,
- 120: PROXIMAL SHAFT,
- 122: INTERMEDIATE SHAFT,
- 124: DISTAL SHAFT,
- 130: BALLOON,
- 140: INNER TUBE SHAFT,
- 142: OPENING PORTION,
- 150: GUIDE WIRE,
- 152: GUIDE WIRE PORT,
- 200: COATING DEVICE,
- 210: CHAMBER,
- 212: BASE,
- 214: MAIN FRAME,
- 215: SUPPORT FRAME,
- 216: DUCT,
- 218: AIR CONDITIONING DEVICE,
- 220: HOLDING TOOL,
- 222: BASE PORTION,
- 224: CHUCKING PORTION,
- 226: MOTOR,
- 228: MANDREL,
- 230: MOVING DEVICE,
- 231: X-DIRECTION MOVING MECHANISM,
- 233: TRAVELING RAIL,
- 234: X-DIRECTION MOVING TABLE,
- 236: Y-DIRECTION MOVING MECHANISM,
- 237: TRAVELING RAIL,
- 238: Y-DIRECTION MOVING TABLE,
- 239: MOTOR,
- 240: DRUG COATING HEAD,
- 245: PRIMER COATING HEAD,
- 252: DISPENSER,
- 253: VERTICAL TABLE,
- 254: MOTOR,
- 255: CYLINDER PORTION,
- 256: PISTON PORTION,
- 257: DRIVE UNIT,
- 258: BRACKET,
- 262: NOZZLE UNIT,
- 264: ATTACHMENT MEMBER,
- 266: NOZZLE,
- 270: FIRST POSITION INFORMATION ACQUISITION DEVICE,
- 272: BRACKET,
- 274: CAMERA UNIT,
- 280: SECOND POSITION INFORMATION ACQUISITION DEVICE,
- 282: BRACKET,
- 284: LASER DISPLACEMENT SENSOR,
- 290: CONTROL UNIT,
- D: LENGTH DIFFERENCE,
- G: GAP,
- T: THICKNESS OF RECOATING LAYER,
- S: AXIAL DIRECTION,
- θ: TILTING ANGLE.

## Claims

1. A stent comprising:
an annular body that is configured to include a waved strut having a bending portion and multiple main strut portions,
wherein only an outer surface of the main strut portion and a side surface of an end portion of the main strut portion adjacent to the bending portion are coated with a drug except the bending portion.

2. The stent according to Claim 1,
wherein the drug is loaded by a polymer so as to configure a coating layer.

3. The stent according to Claim 2,
wherein a side surface of both end portions of the main strut portion is coated with the drug.

4. The stent according to Claim 2 or 3,
wherein a thickness of the drug coating layer in the end portion of the main strut portion gradually decreases toward the bending portion.

5. The stent according to any one of Claims 2 to 4,
wherein the polymer is a biodegradable polymer.

6. The stent according to Claim 5,
wherein the biodegradable polymer is polylactic acid, polyglycolic acid, or a copolymer of lactic acid and glycolic acid.

7. The stent according to any one of Claims 2 to 6,
wherein the annular body is arrayed at multiple locations along an axial direction of the stent,
wherein the strut further has a link portion for integrating the adjacent annular bodies with each other, and
wherein an outer surface of the main strut portion and a side surface of an end portion of the main strut portion adjacent to the bending portion are coated with the drug except the link portion and the bending portion.

8. The stent according to any one of Claims 1 to 7,
wherein a primer coating layer is arranged between the outer surface of the strut and the drug coating layer.

9. A manufacturing method of a stent as defined in any of claims 1 to 8, the method comprising:
A drug-coating process of coating the stent with a drug,
Wherein in the drug-coating process, only an outer surface of the main strut portion and a side surface of an end portion of the main strut portion adjacent to the bending portion are coated with the drug except the bending portion.

## Patentansprüche

1. Stent, umfassend;
einen ringförmigen Körper, der konfiguriert ist, um eine gewellte Strebe mit einem Biegeabschnitt und mehreren Hauptstrebeabschnitten einzuschließen,
wobei nur eine äußere Oberfläche des Hauptstrebeabschnitts und eine seitliche Oberfläche eines Endabschnitts des Hauptstrebeabschnitts, benachbart zu dem Biegeabschnitt, mit einem Wirkstoff beschichtet sind, mit Ausnahme des Biegeabschnitts.

2. Stent gemäß Anspruch 1,
wobei der Wirkstoff durch ein Polymer so beladen ist, um eine Beschichtungsschicht zu konfigurieren.

3. Stent gemäß Anspruch 2,
wobei eine seitliche Oberfläche von beiden Endabschnitten des Hauptstrebeabschnitts mit dem Wirkstoff beschichtet ist.

4. Stent gemäß Anspruch 2 oder 3,
wobei eine Dicke der Wirkstoff-Beschichtungsschicht im Endabschnitt des Hauptstrebeabschnitts in Richtung des Biegeabschnitts graduell abnimmt.

5. Stent gemäß irgendeinem der Ansprüche 2 bis 4,
wobei das Polymer ein biologisch abbaubares Polymer ist.

6. Stent gemäß Anspruch 5,
wobei das biologisch abbaubare Polymer Polymilchsäure, Polyglykolsäure oder ein Copolymer aus Milchsäure und Glykolsäure ist.

7. Stent gemäß irgendeinem der Ansprüche 2 bis 6,
wobei der ringförmige Körper an mehreren Stellen entlang einer axialen Richtung des Stents angeordnet ist,
wobei die Strebe ferner einen Verbindungsabschnitt zum Integrieren der benachbarten ringförmigen Körper miteinander aufweist, und
wobei eine äußere Oberfläche des Hauptstrebeabschnitts und eine seitliche Oberfläche eines Endabschnitts des Hauptstrebeabschnitts benachbart zu dem Biegeabschnitt mit dem Wirkstoff beschichtet sind, mit Ausnahme des Verbindungsabschnitts und des Biegeabschnitts.

8. Stent gemäß irgendeinem der Ansprüche 1 bis 7,
wobei eine Primer-Beschichtungsschicht zwischen der äußeren Oberfläche der Strebe und der Wirkstoff-Beschichtungsschicht angeordnet ist.

9. Herstellungsverfahren eines Stents wie in irgendeinem der Ansprüche 1 bis 8 definiert, das Verfahren umfassend:
ein Wirkstoff-Beschichtungsverfahren zum Beschichten des Stents mit einem Wirkstoff,
wobei bei dem Wirkstoff-Beschichtungsverfahren nur eine äußere Oberfläche des Hauptstrebeabschnitts und eine seitliche Oberfläche eines Endabschnitts des Hauptstrebeabschnitts, benachbart zu dem Biegeabschnitt, mit dem Wirkstoff beschichtet sind, mit Ausnahme des Biegeabschnitts.

## Revendications

1. Stent comprenant :
un corps annulaire qui est configuré pour inclure une entretoise sinueuse ayant une partie incurvée et de multiples parties principales d'entretoise,
dans lequel seulement une surface extérieure de la partie principale d'entretoise et une surface latérale d'une partie d'extrémité de la partie principale d'entretoise adjacente à la partie incurvée sont revêtues d'un médicament à l'exception de la partie incurvée.

2. Stent selon la revendication 1,
dans lequel le médicament est chargé par un polymère de manière à configurer une couche de revêtement.

3. Stent selon la revendication 2,
dans lequel une surface latérale des deux parties d'extrémité de la partie principale d'entretoise est revêtue du médicament.

4. Stent selon la revendication 2 ou 3,
dans lequel une épaisseur de la couche de revêtement par médicament dans la partie d'extrémité de la partie principale d'entretoise diminue progressivement vers la partie incurvée.

5. Stent selon l'une quelconque des revendications 2 à 4,
dans lequel le polymère est un polymère biodégradable.

6. Stent selon la revendication 5,
dans lequel le polymère biodégradable est de l'acide polylactique, de l'acide polyglycolique, ou un copolymère d'acide lactique et d'acide glycolique.

7. Stent selon l'une quelconque des revendications 2 à 6,
dans lequel le corps annulaire est agencé en de multiples endroits le long d'une direction axiale du stent,
dans lequel l'entretoise comporte en outre une partie de liaison pour intégrer les corps annulaires adjacents les uns avec les autres, et
dans lequel une surface extérieure de la partie principale d'entretoise et une surface latérale d'une partie d'extrémité de la partie principale d'entretoise adjacente à la partie incurvée sont revêtues du médicament à l'exception de la partie de liaison et de la partie incurvée.

8. Stent selon l'une quelconque des revendications 1 à 7,
dans lequel une couche de revêtement par apprêt est agencée entre la surface extérieure de l'entretoise et la couche de revêtement par médicament.

9. Procédé de fabrication d'un stent selon l'une quelconque des revendications 1 à 8, le procédé comprenant :
un processus de revêtement par médicament consistant à revêtir le stent d'un médicament,
dans lequel, dans le processus de revêtement par médicament, seulement une surface extérieure de la partie principale d'entretoise et une surface latérale d'une partie d'extrémité de la partie principale d'entretoise adjacente à la partie incurvée sont revêtues du médicament à l'exception de la partie incurvée.
